# EUROPEAN PATENT APPLICATION

(11) **EP 0 608 511 A2**
(43) Date of publication of application: **03.08.1994**
(21) Application number: 93119208.2
(22) Date of filing: 29.11.1993
(51) Int. Cl.: A61K 31/00, A61K 31/685

(54) **Utilization of lysolecithins in the treatment of male impotence and alopecia and the relevant compositions suitable for such use**

(30) Priority: 03.12.1992 IT MI922766
(71) Applicant: Arena, Barbara, I-50052 Monza (MI) (IT); Guadagnini, Giuseppe, I-22060 Carimate (CO) (IT)
(72) Inventor: Arena, Barbara, I-50052 Monza (MI) (IT); Guadagnini, Giuseppe, I-22060 Carimate (CO) (IT)
(74) Representative: Trupiano, Roberto

(57) **Abstract**

Compositions based on Lysolecithins are advantageously used in the treatment of male impotence and alopecia and are exempt from adverse side reactions.

## Description

### OBJECT OF THE INVENTION

Object of the present invention are the utilization of Lysolecithins in the treatment of male impotence and alopecia and the Lysolecithin-based compositions suitable for such use.

### PRIOR ART

Lysolecithins or Lysophosphatidylcholines, which are basic components of the cell membrane, are natural compositions of animal or vegetal origin.

Lysolecithins are obtained by hydrolysis in beta position by Phospholypasis A₂ from the corresponding Lecithins of Phosphatidylcholines. Having a good emulsionating power and solubilizing properties, Lysolecithins are utilized mainly in the cosmetic field, as they permit to obtain stable emulsions, to be used for instance for the preparation of creams, milky lotions, ointments, besides being solubilizing agens for after-shave lotions, hair lotions and lotions in general. Besides, they are also utilized for skin and hair care and in soaps. Some "Lysolecithin-like" compositions are also described as being potential carcinostatic agents (Patent JP 88006554) and in general possible applications in the immune-oncologic field are suggested for Lysolecithins and/or compositions obtained from them.

### PURPOSES OF THE INVENTION

Purpose of the present invention is to provide a new drug utilizable for the treatment of male impotency.

Another purpose of the present invention is to provide a new drug utilizable for the tretament of alopecia. Still another purpose of this invention is to provide compositions suitable to be utilized for the treatment of male impotence and alopecia and that are exempt from adverse side reactions.

### DESCRIPTION OF THE INVENTION

These and still other purposes and related advantages which shall be made clear by the following description are reached by a product constituted by Lysolecithin having the following general formula: 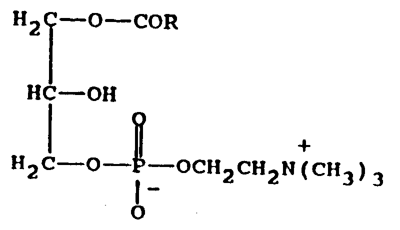
where R are the known characteristic functionalizations of Phospfatidylcholines, and in particular saturated and/or unsaturated fat acids, which product is utilized for the treatment of male impotence and for the treatment of alopecia. In fact, it has been observed that the administration of suitably formulated Lysolecithins causes the dilatation of the cavernous vessels of the penis, facilitating erection, and in the case of alopecia, it activates blood circulation in the scalp, facilitating hair growth. Lysolecithins, according to the present invention, are administered in compositions which comprise, besides excipients and additives of a known type, compounds which are provided with antioxidant characteristics such as, for instance, tocopherol, nordihydroguaiaretic acid, ascorbic acid, butyl-hydroxyanisole and the like, which inhibit the potential chemical degradation characteristic of Lysolecithins, and permit the application of said compositions or formulations, avoiding adverse side reactions, such us local irritations, allergies and the like. According to the present invention, said compositions or formulations are characterized by a content of Lysolecithins comprised between 0.1% and 20%, preferably 0.1 and 10%. Said formulations and compositions containing Lysolecithins, when utilized in the treatment of male impotence or alopecia, are exempt from the adverse side reactions that are characteristic of the utilization of Lysolecithins as such when used in high doses, or in particular experimental conditions, said adverse side reactions being due to cytolytic reactions, and consequently to damages of a cellular type. The drug containing Lysolecithins according to this invention permits therefore to obtain a significant therapeutic activity in the treatment of male impotence and alopecia, allowing the local administration of Lysolecithins in concentrations such as to develop said therapeutic activity without incurring any adverse side reaction. Always according to the present invention, said compositions or formulations containing Lysolecithin are advantageous utilized as condom lubricants, so as to associate the necessary lubricant activity to the therapeutical activity.

Always according to this invention, said compositions or formulations may comprise many additives and excipients of a known type, in amounts variable according to the characteristics required of the final product, as described in the hereunder illustrated examples, which are given by way of indication with no limitations for the protection scope of this invention.

### EXAMPLE 1. Oil/water creamy emulsion

- Soja lysolecithin 5.0 g
- Iso-octilstearate 10.0 g
- Vaseline oil 5.0 g
- Cetylic alcohol 3.0 g
- Polyoxyethylensorbitan monoleate 2.5 g
- Tocopherols 0.5 g
- Carbomer 0.4 g
- Paraseptics 0.2 g
- Distilled water q.s. to 100 g

### EXAMPLE 2. Water/oil creamy emulsion

- Soja lysolecithin 7.5 g
- Tocopherols 0.5 g
- Vaseline 10.0 g
- Vaseline oil 10.0 g
- Sorbitan-monolaurate 2.5 g
- Sorbitan-monostearate 2.5 g
- Conservatives 0.2 g
- Distilled water q.s. to 100 g

### EXAMPLE 3. Milky solution

- Lysolecithin 1.0 g
- Alpha-tocopherol acetate 0.1 g
- Iso-octilstearate 1.0 g
- Vaseline oil 0.5 g
- Polyoxyethylensorbitan monolaurate 0.2 g
- Paraseptics 0.2 g
- Distilled water q.s. to 100 g

### EXAMPLE 4. Solution for the treatment of alopecia

- Lysolecithin 2.5 g
- Tocopherol 2.25 g
- Ethyl alcohol 25.0 g
- Vegetable extracts 0.5 g
- Distilled water q.s. to 100 g

### EXAMPLE 5. Solution for the treatment of alopecia

- Lysolecithin 2.5 g
- Tocopherol 0.2 g
- Triethanolamine laurylsulfate 20.0 g
- Alkylamidobetaine 10.0 g
- Laurylether sodium sulfate 10.0 g
- Conservatives 0.4 g
- Distilled water q.s. to 100 g

The muscle relaxant activity of the cavernous body in the rabbit has been determined acording to the method described by IGNARRO et al. Biochem. Biophys. Res. Commun. 170,843 (1990).

By way of example, Table 1 shows the per cent activity on the rabbit cavernous body of Lysolecithyn suitable vehiculated to make treatment possible; the per cent activity of vehicle only is considered as a reference, treatment conditions being equal. The values shown are a mean of the values obtained from the repetition of 5 tests for each treatment group.

**TABLE 1**

| Product | Muscle relaxating % activity on rabbit cavernous body |
|---|---|
| - Vehiculated Lysolectithin | 20 |
| - Vehicle | 0 |

When locally applied on the shaven skin of the guinea pig or in the eye of the rabbit, the product obtained as described in Example 1 and Example 2, according to the present invention, proved to be very well tolerated even if used at centrations remarkably higher than those that have proved to be efficacious in the specific activity tests.

The efficaciousness and tolerability of the product obtained as described in Example 4 and Example 5, according to the present invention, have been evaluated in patients suffering from alopecia according to the method described by A.C.DE GROOT et al., Lancet 1, 1019 (1987). The treatment has brought to cosmetically acceptable improvements and its tolerability was excellent.

## Claims

1. Product constituted by Lysolecithin having the following general formula: 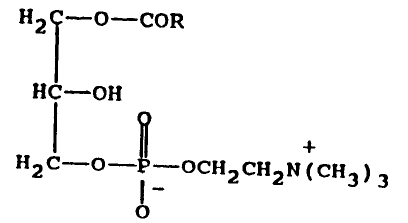 where R are the characteristic functionalizations of phospatidylcholines, and in particular saturated and/or unsaturated fat acids, characterized in that it is utilized for the treatment of male impotence.

2. Product constituted by Lysolecithin according to claim 1, characterized in that it is utilized for the treatment of alopecia.

3. Compositions containing Lysolecithin according to claims 1 and 2, characterized in that said Lysolecithin is associated with compounds having antioxidant properties, said compositions, utilized in the treatment of male impotence and alopecia, being exempt from adverse side reactions.

4. Compositions according to claim 3, characterized in that said compounds having antioxidant properties are chosen among tocopherol, nordihydroguaiaretic acid, ascorbic acid, butylhydroxyanisole.

5. Compositions contaning Lysolecithin according to claims 1 and 2, characterized in that the Lisolecithin content of said compositions is comprised between 0.1 and 20% in weight compared to the total weight of the composition.

6. Compositions containing Lysolecithin according to claims 1 and 2, characterized in that the Lysolecithin content of said compositions is comprised between 1 and 10% in weight compared to the total weight of the composition.

7. Compositions containing Lysolecithin according to claim 3, characterized in that they are used as condom lubricants.
